# NEW EUROPEAN PATENT SPECIFICATION

(11) **EP 2 793 877 B2**
(45) Date of publication and mention of the opposition decision: **04.05.2022**
(45) Mention of the grant of the patent: 15.08.2018
(21) Application number: 12860213.3
(22) Date of filing: 19.12.2012
(51) Int. Cl.: A61K 31/24, A61K 9/68, C07C 229/28, A61P 11/06, A61P 11/00, A61P 27/02, A23G 4/06, A23G 4/20

(54) **CHEWING GUM PRODUCTS CONTAINING [(2-ISOPROPYL-5-METHYL-CYCLOHEXANECARBONYL)-AMINO]-ACETIC ACID ISOPROPYL ESTER**
KAUGUMMIPRODUKTE MIT [(2-ISOPROPYL-5-METHYL-CYCLOHEXANCARBONYL-)AMINO-]ESSIGSÄURE-ISOPROPYLESTER
PRODUITS DE GOMME À MÂCHER CONTENANT DE L'ESTER ISOPROPYLIQUE DE L'ACIDE [(2-ISOPROPYL-5-MÉTHYL-CYCLOHEXANECARBONYL)-AMINO]-ACÉTIQUE

(30) Priority: 21.12.2011 US 201161578714 P; 01.03.2012 US 201261605256 P
(43) Date of publication of application: 29.10.2014
(73) Proprietor: WM. Wrigley Jr. Company, Chicago, IL 60642 (US)
(72) Inventor: JOHNSON, Sonya S., La Grange Highlands, Illinois 60525 (US); SHELDON, Gloria T., Valparaiso, Indiana 46385 (US)
(74) Representative: Dehns
(86) International application number: PCT/US2012/070539
(87) International publication number: WO 2013/096405

(56) References cited:
- WO-A1-94/21135
- WO-A1-2005/082154
- WO-A1-2008/056119
- WO-A1-2011/159927
- WO-A1-2011/159935
- WO-A1-2012/076831
- US-A- 4 136 163
- US-A1- 2007 077 331
- US-A1- 2007 221 236
- US-A1- 2007 248 717
- US-A1- 2008 227 857
- US-A1- 2010 076 080
- US-A1- 2011 082 204
- US-A1- 2011 082 204
- US-A1- 2012 251 461

## Description

### BACKGROUND OF THE INVENTION

The present invention relates to chewing gum. More specifically, this invention relates to formulations for chewing gum compositions containing [(2-isopropyl-5-methyl-cyclohexanecarbonyl)-amino]-acetic acid isopropyl ester, from herein called Gly-OiPr. More particularly, the invention relates to producing chewing gum containing [(2-isopropyl-5-methyl-cyclohexanecarbonyl)-amino]-acetic acid isopropyl ester as a physiological cooling agent.

Many individuals who chew chewing gum desire that the gum have a refreshing taste, and most typically this includes a cooling sensation. Peppermint oil is frequently used to create a "cooling" in oral products such as toothpaste, mouthwash, chewing gum, candy and other food products. Peppermint oil generally comprises about 45% menthol, about 20% menthone, about 5% menthyl acetate, about 5% eucalyptol and many other constituents. Peppermint oil is even used in non-peppermint products, such as spearmint or wintergreen flavored products, in order to create this desired cooling effect. However, peppermint notes are then found in the resulting non-peppermint flavored products.

Menthol is also known for its physiological cooling effect on the skin and mucous membranes of the mouth. Being a major constituent of peppermint oil, menthol has been used extensively in foods, beverages, dentifrices, mouthwashes, toiletries, lotions and the like. The disadvantages of using menthol, however, are its strong minty odor and the harsh notes it imparts to compositions in which it is found.

Several known compounds have what can be characterized as a "cooling" activity, and are referred to in the art as "physiological cooling agents." Physiological cooling agents are perceived as cold or cool when contacted with the human body and, in particular, with the mucous membranes of the mouth, nose and throat. Several of these compounds have been suggested for use in confectionery products, including chewing gum.

However, while these physiological cooling agents are less objectionable than menthol, when they are used at high levels to provide desirable refreshing coolness, they also have objectionable flavor characteristics, such as bitterness, biting and sharpness along with off tastes.

It would be desirable to provide a chewing gum with a higher level of a cooling sensation, but without using materials that manifest the unwanted harshness or flavor characteristics that come from adding menthol. It would also be desirable to provide a clean, high-quality flavor chewing gum with a good cooling and less bitterness.

EP2649046, which is prior art under Article 54(3) EPC, discloses in an example "Study 4", a wafer of Wrigley spearmint chewing gum having Gly-O-iPr spread thereon. The Gly-O-iPr is not evenly dispersed throughout the chewing gum composition.

US2007/077331 discloses cooling compositions comprising a menthyl ester and a second cooling agent. WO2005/082154 discloses confections comprising menthyl glutarate and one or more other physiological cooling agents selected from /-isopulegol, p-menthane-3,8-diol, or mixtures thereof.

US2011/082204 discloses a towlette containing a cooling compound. In an embodiment the cooling compound may be Gly-OiPr.

### SUMMARY OF THE INVENTION

Chewing gum compositions have been invented that provide a high level of refreshing taste with significant breath freshening without objectionable flavor characteristics. In a first aspect, the invention is a chewing gum composition comprising gum base, flavor, sweetening agent, and 0.01% to 0.4% Gly-OiPr, wherein the Gly-OiPr is evenly dispersed throughout the chewing gum composition.

In another aspect, the invention includes a chewing gum composition which comprises gum base, flavor, at least one sweetening agent selected from the group consisting of sugars, sugar alcohols, and mixtures thereof, and a cooling agent other than Gly-OiPr in a amount to impart cooling to said gum at a level in the range found in typical commercial gum products, the improvement comprising dispersing 0.01% to 0.4% Gly-OiPr by weight of the total gum composition throughout the chewing gum composition, whereby the cooling and breath freshening are enhanced.

In another aspect, the invention includes a chewing gum composition comprising 5% to 95% gum base, 0.1% to 10% flavor, 10% to 90% sweetening agent selected from the group consisting of sugars, sugar alcohols, and mixtures thereof, and 0.01 to 0.4% Gly-OiPr, wherein the Gly-OiPr is evenly dispersed throughout the chewing gum composition.

The use of Gly-OiPr at levels of 0.01% to 0.4% has been surprisingly found to provide a gum composition having high levels of refreshing taste with considerable cooling and breath freshening but without unwanted flavor characteristics as compared to even ethyl ester of N-[[5-methyl-2-(1-methylethyl)-cyclohexyl] carbonyl] glycine (WS-5), which Gly-OiPr is an analogue of WS-5. The Gly-OiPr physiological cooling agent may be used in combination with other physiological cooling agents, and it may be treated to control its release and enhance its shelf life stability. Also, the Gly-OiPr physiological cooling agent may be added as part of a cooling flavor composition, or used in a chewing gum coating. Additional features and advantages of the disclosed embodiments are described in, and will be apparent from, the following Detailed Description and the attached drawings.

### BRIEF DESCRIPTION OF THE DRAWINGS

Figure 1 is a graphical illustration of the average cooling intensity at each minute interval, during 20 minutes of chewing, of a chewing gum of the present invention compared to chewing gum composition containing WS-5.
Figure 2 is a graphical illustration of the average bitterness intensity of solutions with Gly-OiPr compared to solutions with WS-5 at different concentrations.

### DETAILED DESCRIPTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Unless otherwise specified, all percentages herein are weight percentages. Although some terms are referred to in the singular, it is understood that such references may also encompass the plural. For example, although chewing gum coating is referred to in the singular, it is understood that coated chewing gum normally contains multiple layers of coating. Therefore a phrase that refers to "the coating," refers to one or more layers of coating.

Several terms used in the specification and claims have a meaning defined as follows.

In the context of this invention, chewing gum refers to chewing gum, bubble gum and the like.

The breath freshening discussed herein is consumer perceived breath freshening, rather than objectively measured breath freshening.

Physiological cooling agents encompass any number of physiological cooling agents. However, in the context of this invention, the term "physiological cooling agent" does not include traditional flavor-derivatives such as menthol or menthone. Preferred physiological cooling agents do not have a perceptible flavor of their own, but simply provide a cooling effect.

The term "Gly-OiPr" refers to [(2-isopropyl-5-methyl-cyclohexanecarbonyl)-amino]-acetic acid isopropyl ester. There are different isomers of Gly-OiPr that may be used in the present invention and one example is [((1R,2S,5R)-2-isopropyl-5-methyl-cyclohexanecarbonyl)-amino]-acetic acid isopropyl ester. Gly-OiPr has a structure illustrated below:

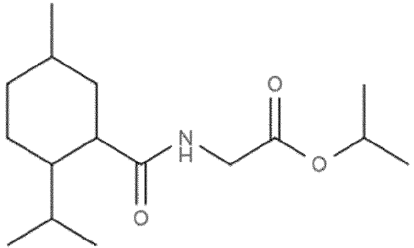

It is recognized that some compositions obtained as Gly-OiPr may not be pure [(2-isopropyl-5-methyl-cyclohexanecarbonyl)-amino]-acetic acid isopropyl ester, but may contain small percentages of impurities that are byproducts of manufacture. Preferably the Gly-OiPr used in the gum compositions of the present invention will have a purity of at least 96% Gly-OiPr. In the present application, Gly-OiPr means 100% pure [(2-isopropyl-5-methyl-cyclohexanecarbonyl)-amino]-acetic acid isopropyl ester. If an impure mixture is used, usage levels may need to be adjusted to take the purity level into account.

Particular examples of physiological cooling agents that may be used along with Gly-OiPr in chewing gums of the present invention include:
1) substituted p menthanes and substituted p-menthane-carboxamides, such as those disclosed in U.S. Patents Nos. 4,060,091; 4,190,643 and 4,136,163, all assigned to Wilkinson Sword;
2) acyclic carboxamides, such as those disclosed in U.S. Patents Nos. 4,296,255; 4,230,688; and 4,153,679; all assigned to Wilkinson Sword, especially N-ethyl-p-menthane-3-carboxamide (WS-3, FEMA 3455));
3) acyclic carboxamides (e.g., N,2,3-trimethyl-2-isopropyl butanamide (WS-23, FEMA 3804));
4) Other compounds from Wilkinson Sword, including substituted cyclohexanamides, substituted cyclohexane carboxamides, substituted ureas and sulphonamides, and substituted menthanols;
5) hydroxymethyl and hydroxyethyl derivatives of p-menthane;
6) menthyl succinate (FEMA 3810);
7) 2-mercapto-cyclo-decanone;
8) 2-isopropanyl-5-methylcyclohexanol (hereinafter "isopulegol", FEMA 2962);
9) hydroxycarboxylic acids with 2-6 carbon atoms;
10) menthone glycerol ketals (FEMA 3807);
11) menthol propylene glycol carbonate (FEMA 3806);
12) menthol ethylene glycol carbonate (FEMA 3805);
13) 3-I-menthoxypropane-1,2-diol (FEMA 3784);
14) menthyl lactate; (FEMA 3748);
15) monomenthyl glutarate (FEMA 4006); and
16) ethyl ester of N-[[5-methyl-2-(1-methylethyl)-cyclohexyl] carbonyl] glycine, also called [(ethoxycarbonyl) methyl]-p-menthane-3-carboxamide (WS-5, FEMA 4309) with a structure illustrated below:

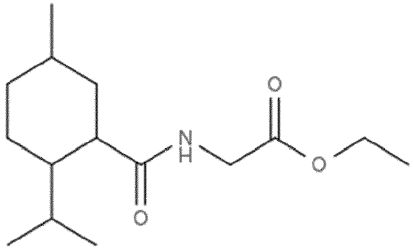

While any of the above-disclosed physiological cooling agents may be included in chewing gum, the physiological cooling agents that are presently preferred to be included with the Gly-OiPr are menthyl succinate; menthyl lactate; 3-I-menthoxypropane-1,2-diol; N-substituted p-menthane carboxamides; acyclic carboxamides; menthone glycerol ketals, menthyl glutarate, /-isopulegol, WS-5 and mixtures thereof. The concentration of physiological cooling agent will depend on the intensity of the physiological cooling agent (for example, WS-5 has a higher cooling effect than WS-3 and WS-23 at the same concentration levels) and the desired cooling effect. The additional physiological cooling agent, when used, will preferably be present at 0.01% to 0.5% of the chewing gum product, preferably with no one of the additional physiological cooling agents comprising more that 0.4% of the gum product. Depending on the level of Gly-OiPr that is used, the level of the other physiological cooling agents (when used) may be between 0.05% and 0.4%, or even between 0.1% and 0.3% of the chewing gum product.

The present invention contemplates that optionally sensates like warming agents and/or tingling agents may also be included in the chewing gum. Warming agents provide to the user a sensation of warmth and tingling agents provide to the user a sensation of tingling. Examples of warming and tingling agents can be found in U.S. Patent No. 6,890,567.

The present invention contemplates that Gly-OiPr and optional additional physiological cooling agents may be added to the flavor used to make the chewing gum. On the other hand, the flavor and cooling agents may be added separately anywhere within the manufacturing process for making a chewing gum. In addition, physiological cooling agents including Gly-OiPr may be encapsulated to modify their release rate from chewing gum. The physiological cooling agents or sweeteners may be fully or partially encapsulated with water-soluble or water-insoluble materials. Some encapsulation procedures include spray drying, spray chilling, fluid-bed coating, coacervation, extrusion, and other agglomerating and encapsulating techniques. Encapsulation materials include acrylic polymers and copolymers, carboxyvinyl polymer, polyamides, polystyrene, polyvinyl acetate, polyvinyl acetate phthalate, polyvinyl pyrrolidone, waxes, shellac, zein, agar, alginates, a wide variety of cellulose derivatives like ethyl cellulose and hydroxypropylmethyl cellulose, dextrin, gelatin, modified starches, acacia, maltodextrin, gum arabic, guar gums, locust bean gum, carrageenan, and mixtures thereof. Additionally, the physiological cooling agents may also be adsorbed onto an inert or water-insoluble material such as silicas, silicates, pharmasorb clay, sponge-like beads or microbeads, amorphous carbonates and hydroxides, including aluminum and calcium lakes. The cooling agents including Gly-OiPr may be modified in a multiple step process comprising any of the techniques noted.

These flavors include any flavor which is of food acceptable quality commonly known in the art such as essential oils, synthetic flavors or mixtures thereof. Such flavors include, but are not limited to, oils derived from plants and fruits such as citrus oils, fruit essences, peppermint oil, spearmint oil, eucalyptus, other mint oils, clove oil, oil of wintergreen, cinnamic aldehyde, anise, spice flavors, and the like. Flavors that are very strong, such as menthol flavors, are also contemplated in this invention. Preferred flavors include cooling flavors such as peppermint, eucalyptus, menthol, wintergreen and fruity-mint; non-cooling flavors such as spearmint and cinnamon; and combinations thereof. In some embodiments, the chewing gum products will include menthol, and may include 0.01% to 2.0% menthol, preferably less than 0.5%.

Artificial flavor components are also contemplated by the present invention. Those of ordinary skill in the art will recognize that natural and artificial flavors may be combined in any sensorially acceptable blend. All such flavors and blends are contemplated by the present invention.

The flavor may be added to the chewing gum formula in an amount such that it will contain from 0.1% to 10% flavor, preferably from 0.2% to 4.0% flavor, and most preferably 0.5% to 2% flavor.

Physiological cooling agents in a liquid form may be added directly to a chewing gum formulation in its liquid form or may be combined with flavors or with other solvents such as alcohol, glycerin, propylene glycol, flavor solvents, emulsifiers, or vegetable oils. Physiological cooling agents in crystalline or powder form may also be added directly to a chewing gum formulation in its powder form or may be combined with other powdered bulking agents such as sugars, polyols, and other types of powdered ingredients. In some cases physiological cooling agents may be emulsified in flavor/water compositions or oil/water compositions. Most importantly, because of the low level of usage, the physiological cooling agents need to be evenly dispersed throughout the chewing gum composition.

In most instances, liquid physiological cooling agents may be combined and readily added directly to a gum or confectionery formulation. In other instances, crystalline or powder physiological cooling agents as well as menthol may be dissolved in other liquid physiological cooling agents and the combinations readily added directly to a gum or confectionery formulation. It is also known that some crystalline physiological cooling agents as well as menthol may be combined to form eutectic mixtures which have a lower melting point than the individual crystalline cooling agents themselves. As a result, mixtures of some crystalline physiological cooling agents can be melted, blended together, and remain liquid at or near room temperature and can then be added directly to a chewing gum or confectionery formulation. Combinations of menthol with physiological cooling agents such as menthyl lactate, menthyl succinate, p-menthane carboxamides like WS-3, acyclic carboxamides like WS-23, can be melted together and used readily in liquid form in product formulations.

The Gly-OiPr may be used in sugarless gum formulations and may also be used in a sugar chewing gum. The Gly-OiPr may be used in either regular chewing gum or bubble gum. The Gly-OiPr may also be used in a low-moisture gum.

The chewing gum composition of the present invention follows the general pattern outlined below. In general, a chewing gum composition typically contain a chewable gum base portion which is essentially free of water and is water-insoluble, a water-soluble bulk portion and flavors which are typically water insoluble. The water-soluble portion dissipates with a portion of the flavor over a period of time during chewing. The gum base portion is retained in the mouth throughout the chew.

The insoluble gum base generally comprises elastomers, elastomer solvents, plasticizers, waxes, emulsifiers and inorganic fillers. Plastic polymers, such as polyvinyl acetate, which behave somewhat as plasticizers, are also often included. Other plastic polymers that may be used include polyvinyl laureate, polyvinyl alcohol and polyvinyl pyrrolidone.

Elastomers may include polyisobutylene, butyl rubber, (isobutylene-isoprene copolymer) and styrene butadiene rubber, as well as natural latexes such as chicle. Elastomer solvents are often resins such as terpene resins and rosin esters. Plasticizers, sometimes called softeners, are typically fats and oils, including tallow, hydrogenated and partially hydrogenated vegetable oils, and cocoa butter. Commonly employed waxes include paraffin, microcrystalline and natural waxes such as beeswax and carnauba. Microcrystalline waxes, especially those with a high degree of crystallinity, may be considered bodying agents or textural modifiers.

According to the preferred embodiment of the present invention, the insoluble gum base constitutes between 5% to 95% by weight of the gum. More preferably the insoluble gum base comprises between 10% and 50% by weight of the gum and most preferably 20% to 35% by weight of the gum.

The gum base typically also includes a filler component. The filler component may be calcium carbonate, magnesium carbonate, talc, dicalcium phosphate or the like. The filler may constitute between 5% and 60% by weight of the gum base. Preferably the filler comprises 5% to 50% by weight of the gum base.

Gum bases typically also contain softeners including glycerol monostearate and glycerol triacetate. Gum bases may also contain optional ingredients such as antioxidants, colors, and emulsifiers. The present invention contemplates employing any commercially acceptable gum base.

The water-soluble portion of the chewing gum may further comprise softeners, sweeteners, flavors, fillers, physiological cooling agents and combinations thereof. The sweeteners often fulfill the role of bulking agents in the gum. The bulking agents typically comprise 5% to 95% of the gum composition.

Softeners are added to the chewing gum in order to optimize the chewability and mouth feel of the gum. Softeners, also known in the art as plasticizers or plasticizing agents, generally constitute between 0.5% to 15% of the chewing gum. Softeners contemplated by the present invention include glycerin, lecithin and combinations thereof. Further, aqueous sweetener solutions such as those containing sorbitol, hydrogenated starch hydrolysate, corn syrup and combinations thereof may be used as softeners and binding agents in gum.

As mentioned above, the Gly-OiPr and optional additional physiological cooling agents of the present invention may be used in sugarless gum formulations. However, formulations containing sugar are also within the scope of the invention. Sugar sweeteners generally include saccharide-containing components commonly known in the chewing gum art which comprise, but are not limited to, sucrose, dextrose, maltose, dextrin, dried invert sugar, fructose, galactose, corn syrup solids and the like, alone or in any combination.

The Gly-OiPr and optional additional physiological cooling agents of the present invention can also be used in combination with sugarless sweeteners. Generally sugarless sweeteners include components with sweetening characteristics but which are devoid of the commonly known sugars and comprise, but are not limited to, sugar alcohols such as sorbitol, hydrogenated isomaltulose, mannitol, xylitol, lactitol, erythritol, hydrogenated starch hydrolysate, maltitol and the like alone or in any combination

Depending on the particular sweetness release profile and shelf-stability needed, free or encapsulated high-intensity sweeteners may be used in the chewing gum composition, or may be used in a coating applied to centers made from those gum compositions. High-intensity sweeteners, preferably aspartame, may be used at levels from 0.01% to 3.0%. Encapsulated aspartame is a high intensity sweetener with improved stability and release characteristics, as compared to free aspartame. Free aspartame can also be added, and a combination of some free and encapsulated aspartame is preferred when aspartame is used. Other high intensity sweeteners that may be used in the gum center are: saccharin, Thaumatin, alitame, saccharin salts, sucralose, Stevia, and acesulfame K. Overall, the chewing gum composition will preferable comprise 0.5% to 90% sweetening agents. Most typically the sweetening agents will comprises at least one bulk sweetener and at least one high-intensity sweetener.

Optional ingredients such as colors, emulsifiers and pharmaceutical agents may also be added as separate components of the chewing gum composition, or added as part of the gum base.

Aqueous syrups, such as corn syrup and hydrogenated corn syrup may be used, particularly if their moisture content is reduced. This can preferably be done by coevaporating the aqueous syrup with a plasticizer, such as glycerin or propylene glycol, to a moisture content of less than 10%. Preferred compositions include hydrogenated starch hydrolysate solids and glycerin. Such syrups and their methods of preparation are discussed in detail in U.S. Patent No. 4,671,967.

A preferred method of manufacturing chewing gum according to the present invention is by sequentially adding the various chewing gum ingredients to any commercially available mixer known in the art. After the ingredients have been thoroughly mixed, the gum is discharged from the mixer and shaped into the desired form such as by rolling into sheets and cutting into sticks, extruding into chunks, or casting into pellets.

Generally, the ingredients are mixed by first melting the gum base and adding it to the running mixer. The base may also be melted in the mixer itself. Color or emulsifiers may also be added at this time, along with syrup and a portion of the bulking agent. Further portions of the bulking agent may then be added to the mixer. A flavoring agent is typically added with the final portion of the bulking agent. The Gly-OiPr physiological cooling agent may be mixed with the flavor composition of the present invention and preferably added as part of the flavor addition. If the Gly-OiPr is encapsulated to modify its release rate, it will preferably be added after the final portion of bulking agent and flavor has been added. The entire mixing procedure typically takes from five to twenty minutes, but longer mixing times may sometime be required. Those skilled in the art will recognize that many variations of the above described procedures may be followed.

If formed into pellets or balls, the chewing gum composition can be coated. The coating is initially present as a liquid syrup which contains from 30% to 85% or 30 to 80% sugars or sugar alcohols, and from 15% to 70 or 20% to 70% of a solvent such as water. In general, the coating process is carried out in conventional panning equipment. Gum center tablets to be coated are placed into the panning equipment to form a moving mass.

The material or syrup which will eventually form the coating is applied or distributed over the gum center tablets. Flavors may be added before, during and after applying the syrup to the gum centers. Once the coating has dried to form a hard surface, additional syrup additions can be made to produce a plurality of coatings or multiple layers of coating. The Gly-OiPr may be added in the coating from 0.01% to 0.4% by weight of the coating or from 0.05% to 0.3% or from 0.07% to 0.2% of the coating.

In the panning procedure, syrup is added to the gum center tablets at a temperature range of from 100°F to 240°F. Preferably, the syrup temperature is from 140°F to 200°F. Most preferably, the syrup temperature should be kept constant throughout the process in order to prevent the polyol in the syrup from crystallizing. The syrup may be mixed with, sprayed upon, poured over, or added to the gum center tablets in any way known to those skilled in the art.

In another embodiment, a soft coating is formed by adding a powder coating after a liquid coating. The powder coating may include natural carbohydrate gum hydrolysates, maltodextrin, gelatin, cellulose derivatives, starches, modified starches, sugars, sugar alcohols, natural carbohydrate gums and fillers like talc and calcium carbonate.

Each component of the coating on the gum center may be applied in a single layer or in a plurality of layers. In general, a plurality of layers is obtained by applying single coats, allowing the layers to dry, and then repeating the process. The amount of solids added by each coating step depends chiefly on the concentration of the coating syrup. Any number of coats may be applied to the gum center tablet. Preferably, no more than 75 coats are applied to the gum center. More preferably, less than 60 coats are applied and most preferably, 30 to 60 coats are applied. In any event, the present invention contemplates applying an amount of syrup sufficient to yield a coated chewing gum product containing 10% to 65% coating. Preferably, the final product will contain from 20% to 50% coating.

Those skilled in the art will recognize that in order to obtain a plurality of coated layers, a plurality of premeasured aliquots of coating syrup may be applied to the gum center. It is contemplated, however, that the volume of aliquots of syrup applied to the gum center may vary throughout the coating procedure.

Once a coating of syrup is applied to the gum center, the syrup is dried in an inert medium. A preferred drying medium comprises air. Preferably, forced drying air contacts the wet syrup coating in a temperature range of from 70°F to 110°F. More preferably, the drying air is in the temperature range of from 80°F to 100°F. The invention also contemplates that the drying air possesses a relative humidity of less than 15 percent. Preferably, the relative humidity of the drying air is less than 8 percent.

The drying air may be passed over and admixed with the syrup coated gum centers in any way commonly known in the art. Preferably, the drying air is blown over and around the syrup coated gum center. If a flavor is applied after a syrup coating has been dried, the present invention contemplates drying the flavor with or without the use of a drying medium.

### EXAMPLES

The following examples of the invention and comparative formulations are provided to illustrate, but not to limit, the invention which is defined by the attached claims. Amounts listed are in weight percent.

Several chewing gum compositions were made, and their compositions are provided in Table 1 below.

**TABLE 1**

| | Comparative Example A | Example 1 |
|---|---|---|
| Sugarless syrup** | 36.38% | 36.38% |
| Sorbitol | 34.56% | 34.56% |
| Gum base | 25.90% | 25.90% |
| Peppermint oil* | 2.22% | 2.22% |
| Gly-OiPr* | | 0.10% |
| WS-5* | 0.10% | |
| Encapsulated high intensity sweeteners | 0.40% | 0.40% |
| Free high intensity sweeteners | 0.44% | 0.44% |
| Total | 100.00% | 100.00% |
| *Gly-OiPr and WS-5 were dissolved in this peppermint oil before adding to gum. | | |
| **Coevaporated aqueous mixture of sorbitol, mannitol, maltitol, and glycerin. | | |

The peppermint oil used in Comparative Examples A and Example 1 naturally contain menthol (from 40% to 50%), menthone (from 20% to 25%), and eucalyptol (from 6% to 7%).

Samples of Comparative Example A and Example 1 were evaluated by a trained group of five panelist, who were given a sensory questionnaire in which they were asked to rate the samples in cooling intensity. They each chewed the samples for 20 minutes, and were asked to rate the cooling intensity of the sample at each minute interval. The results of the cooling intensity test are shown in Table 2.

| TABLE 2 | | |
|---|---|---|
| Average cooling intensity score during 20 minute chew | | |
| | Average Cooling Intensity Score | |
| Time (minutes) | Comparative Example A | Example 1 |
| 0.5 | 4.20 | 3.40 |
| 1 | 4.20 | 3.80 |
| 2 | 4.60 | 4.60 |
| 3 | 4.80 | 5.80 |
| 4 | 5.40 | 6.40 |
| 5 | 5.60 | 6.60 |
| 6 | 5.80 | 6.60 |
| 8 | 5.60 | 6.60 |
| 10 | 5.20 | 6.00 |
| 12 | 4.80 | 5.80 |
| 15 | 4.20 | 5.60 |
| 18 | 4.60 | 5.60 |
| 20 | 4.40 | 5.40 |

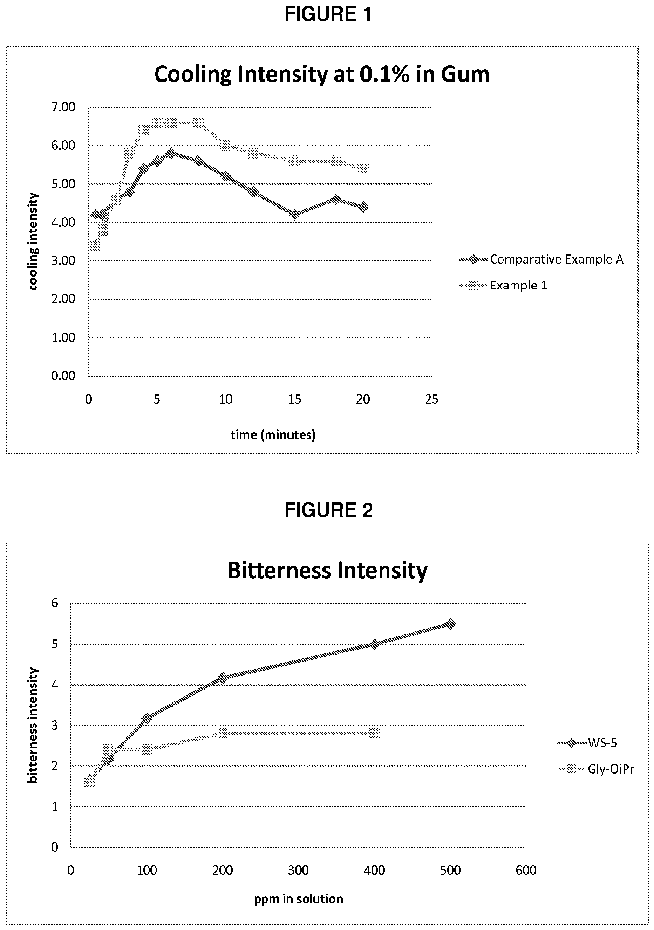

Figure 1 shows the plotted graph of the scores in Table 2. Comparative Example A contains 0.1% of WS-5 in the chewing gum and Example 1 contains 0.1% of Gly-OiPr. Even though the chewing gum formulations are the same between Comparative Example A and Example 1 except for the cooling agent, after minute 2, Example 1 with Gly-OiPr surprisingly sustained a higher level of cooling intensity for the rest of the 20 minute chew as compared to the chewing gum composition with WS-5. Based on this test, the composition with Gly-OiPr was able to sustain a higher level of cooling with the same amount of material compared to WS-5. Therefore, Gly-OiPr has an advantage over WS-5 in that it is possible to achieve the same level of cooling without putting in as much material which can have cost advantages and decreased bitterness as well.

Table 3 and Figure 2 show test results of a trained sensory panel of five to six panelists where the panelists were asked to rate the bitterness intensity of solutions prepared with different concentrations of WS-5 and Gly-OiPr.

| Table 3 | | |
|---|---|---|
| Average bitterness intensity score | | |
| | Average Bitterness Intensity Score | |
| Concentration (ppm) | WS-5 | Gly-OiPr |
| **25** | 1.67 | 1.6 |
| **50** | 2.17 | 2.4 |
| **100** | 3.17 | 2.4 |
| **200** | 4.17 | 2.8 |
| **400** | 5 | 2.8 |
| **500** | 5.5 | |

The sample solutions used in Table 3 and Figure 2 were prepared with 5% sucrose solutions using distilled deionized water with differing concentrations (in ppm) of WS-5 and Gly-OiPr. The results surprisingly show that at higher concentrations of cooling agent in solution, the perceived bitterness intensity for Gly-OiPr is much lower than WS-5. For example, even though the concentration of Gly-OiPr was doubled from 200ppm to 400ppm, the bitterness intensity remained the same. The rate at which the bitterness intensity increases with concentration for Gly-OiPr is much lower than the rate for WS-5, as shown by the graph in Figure 2.

It should be appreciated that the methods and compositions of the present invention are capable of being incorporated in the form of a variety of embodiments, only a few of which have been illustrated and described above. The invention may be embodied in other forms without departing from its spirit or essential characteristics. The described embodiments are to be considered in all respects only as illustrative and not restrictive, and the scope of the invention is, therefore, indicated by the appended claims rather than by the foregoing description. All changes which come within the meaning and range of equivalency of the claims are to be embraced within their scope.

## Claims

1. A chewing gum composition comprising:
a) gum base,
b) flavor,
c) sweetening agent, and
d) 0.01% to 0.4% [(2-isopropyl-5-methyl-cyclohexanecarbonyl)-amino]-acetic acid isopropyl ester (Gly-OiPr) by weight of the chewing gum composition;
wherein the Gly-OiPr is evenly dispersed throughout the chewing gum composition.

2. The chewing gum composition of claim 1 wherein the composition further comprises an additional physiological cooling agent.

3. The chewing gum composition of claim 2 wherein the additional physiological cooling agent is selected from the group consisting of menthyl succinate; menthyl lactate; 3-/-menthoxypropane-1,2-diol; N-substituted p-menthane carboxamides; acyclic carboxamides; menthone glycerol ketals, menthyl glutarate, *l-*isopulegol, ethyl ester of N-[[5-methyl-2-(1-methylethyl)-cyclohexyl] carbonyl] glycine and mixtures thereof.

4. The chewing gum composition of any of claims 1 to 3 wherein the composition further comprises menthol.

5. The chewing gum composition of any one of claims 1 to 4 wherein the sweetening agent comprises: a) at least one sweetening agent selected from the group consisting of sugars, sugar alcohols, and mixtures thereof, and b) at least one high-intensity sweetener.

6. The chewing gum composition of any one of claims 1 to 5 wherein the flavor is selected from the group consisting of mint flavor, spice flavor, and fruit flavor.

7. The chewing gum composition of any one of claims 1 to 6 wherein the composition further comprises a warming agent or tingling agent.

8. The chewing gum composition of claims 1 to 7 wherein the composition comprises 0.05% to 0.3% Gly-OiPr, such as 0.07% to 0.2% Gly-OiPr.

9. The chewing gum composition of any one of claims 7 to 8 wherein the Gly-OiPr is encapsulated.

10. The chewing gum composition of any one of claims 1 to 9 wherein the Gly-OiPr is the isomer [((1R,2S,5R)-2-isopropyl-5-methyl-cyclohexanecarbonyl)-amino]-acetic acid isopropyl ester.

11. A chewing gum composition as claimed in claim 1 comprising:
a) 5% to 95% gum base,
b) 0.1% to 10% flavor,
c) 10% to 90% sweetening agent selected from the group consisting of sugars, sugar alcohols, and mixtures thereof, and
d) 0.01 to 0.4% [(2-isopropyl-5-methyl-cyclohexanecarbonyl)-amino]-acetic acid isopropyl ester (Gly-OiPr)
by weight of the chewing gum composition.

## Patentansprüche

1. Kaugummizusammensetzung, die Folgendes umfasst:
a) Gummibasis,
b) Geschmacksstoff,
c) Süßungsmittel und
d) 0,01 Gew.-% bis 0,4 Gew.-% der Kaugummizusammensetzung [(2-lsopropyl-5-methyl-cyclohexancarbonyl)-amino]-essigsäureisopropylester (Gly-OiPr);
wobei der Gly-OiPr in der Kaugummizusammensetzung gleichmäßig verteilt ist.

2. Kaugummizusammensetzung nach Anspruch 1, wobei die Zusammensetzung weiter ein zusätzliches physiologisches Kühlmittel umfasst.

3. Kaugummizusammensetzung nach Anspruch 2, wobei das zusätzliche physiologische Kühlmittel aus der Gruppe ausgewählt ist, die aus Folgendem besteht: Menthylsuccinat; Menthyllactat; 3-*l*-Menthoxypropan-1,2-diol; N-substituierten p-Menthancarboxamiden; azyklischen Carboxamiden; Menthonglycerinketalen, Menthylglutarat, *l*-Isopulegol, Ethylester von N-[[5-Methyl-2-(1-methylethyl)-cyclohexyl]carbonyl]glycin und Gemischen davon.

4. Kaugummizusammensetzung nach einem der Ansprüche 1 bis 3, wobei die Zusammensetzung weiter Menthol umfasst.

5. Kaugummizusammensetzung nach einem der Ansprüche 1 bis 4, wobei das Süßungsmittel Folgendes umfasst: a) mindestens ein Süßungsmittel, ausgewählt aus der Gruppe bestehend aus Zuckern, Zuckeralkoholen und Gemischen davon, und b) mindestens einen hochintensiven Süßstoff.

6. Kaugummizusammensetzung nach einem der Ansprüche 1 bis 5, wobei der Geschmacksstoff aus der Gruppe ausgewählt ist, die aus Minzgeschmack, Gewürzgeschmack und Fruchtgeschmack besteht.

7. Kaugummizusammensetzung nach einem der Ansprüche 1 bis 6, wobei die Zusammensetzung weiter ein Erwärmungsmittel oder ein Prickelmittel umfasst.

8. Kaugummizusammensetzung nach einem der Ansprüche 1 bis 7, wobei die Zusammensetzung 0,05 % bis 0,3 % Gly-OiPr, wie 0,07 % bis 0,2 % Gly-OiPr umfasst.

9. Kaugummizusammensetzung nach einem der Ansprüche 7 bis 8, wobei der Gly-OiPr verkapselt ist.

10. Kaugummizusammensetzung nach einem der Ansprüche 1 bis 9, wobei der Gly-OiPr das Isomer [((1R,2S,5R)-2-Isopropyl-5-methyl-cyclohexancarbonyl)amino]-essigsäureisopropylester ist.

11. Kaugummizusammensetzung nach Anspruch 1, die Folgendes umfasst:
a) 5 Gew.-% bis 95 Gew.-% Gummibasis,
b) 0,1 Gew.-% bis 10 Gew.-% Geschmacksstoff,
c) 10 Gew.-% bis 90 Gew.-% Süßungsmittel, ausgewählt aus der Gruppe, die aus Zuckern, Zuckeralkoholen und Gemischen davon besteht, und
d) 0,01 Gew.-% bis 0,4 Gew.-% [(2-lsopropyl-5-methyl-cyclohexancarbonyl)-amino]-essigsäureisopropylester (Gly-OiPr) der Kaugummizusammensetzung.

## Revendications

1. Composition de gomme à mâcher comprenant :
a) une base de gomme,
b) un arôme,
c) un agent édulcorant, et
d) 0,01 % à 0,4 % d'ester isopropylique de l'acide [(2-isopropyl-5-méthyl-cyclohexanecarbonyl)-amino]-acétique (Gly-OiPr) en poids de la composition de gomme à mâcher ;
dans laquelle le Gly-OiPr est dispersé de façon homogène dans toute la composition de gomme à mâcher.

2. Composition de gomme à mâcher selon la revendication 1 dans laquelle la composition comprend en outre un agent rafraîchissant physiologique supplémentaire.

3. Composition de gomme à mâcher selon la revendication 2 dans laquelle l'agent rafraîchissant physiologique supplémentaire est choisi parmi le groupe consistant en du succinate de menthyle ; du lactate de menthyle ; du 3-*l*-menthoxypropane-1,2-diol ; des carboxamides de p-menthane N-substitués ; des carboxamides acycliques ; du glycérol cétals de menthone, du glutarate de menthyle, du *l*-isopulégol, de l'ester éthylique de N-[[5-méthyl-2-(1-méthyléthyl)-cyclohexyl]carbonyl]glycine et des mélanges de ceux-ci.

4. Composition de gomme à mâcher selon l'une quelconque des revendications 1 à 3 dans laquelle la composition comprend en outre du menthol.

5. Composition de gomme à mâcher selon l'une quelconque des revendications 1 à 4 dans laquelle l'agent édulcorant comprend : a) au moins un agent édulcorant choisi parmi le groupe consistant en des sucres, des polyols, et des mélanges de ceux-ci, et b) au moins un édulcorant haute intensité.

6. Composition de gomme à mâcher selon l'une quelconque des revendications 1 à 5 dans laquelle l'arôme est choisi parmi le groupe consistant en un arôme de menthe, un arôme d'épices et un arôme de fruits.

7. Composition de gomme à mâcher selon l'une quelconque des revendications 1 à 6 dans laquelle la composition comprend en outre un agent chauffant ou un agent picotant.

8. Composition de gomme à mâcher selon les revendications 1 à 7 dans laquelle la composition comprend de 0,05 % à 0,3 % de Gly-OiPr, par exemple de 0,07 % à 0,2 % de Gly-OiPr.

9. Composition de gomme à mâcher selon l'une quelconque des revendications 7 à 8 dans laquelle le Gly-OiPr est encapsulé.

10. Composition de gomme à mâcher selon l'une quelconque des revendications 1 à 9 dans laquelle le Gly-OiPr est l'isomère de l'ester isopropylique de l'acide [((1R,2S,5R)-2-isopropyl-5-méthyl-cyclohexanecarbonyl)-amino]-acétique.

11. Composition de gomme à mâcher selon la revendication 1 comprenant :
a) 5 % à 95 % de base de gomme,
b) 0,1 % à 10 % d'un arôme,
c) 10 % à 90 % d'un agent édulcorant choisi parmi le groupe consistant en des sucres, des polyols et des mélanges de ceux-ci, et
d) 0,01 % à 0,4 % d'ester isopropylique de l'acide [(2-isopropyl-5-méthyl-cyclohexanecarbonyl)-amino]-acétique (Gly-OiPr)
en poids de la composition de gomme à mâcher.
